(19)

Europäisches Patentamt
European Patent Office
Office européen des brevets

(11) **EP 3 984 517 A1**

(12) **EUROPEAN PATENT APPLICATION**

(43) Date of publication:
**20.04.2022  Bulletin 2022/16**

(21) Application number: **20202539.1**

(22) Date of filing: **19.10.2020**

(51) International Patent Classification (IPC):
*A61K 8/02* (2006.01)   *A61K 8/24* (2006.01)
*A61Q 11/00* (2006.01)   *A61K 33/42* (2006.01)
*A61K 33/16* (2006.01)   *A61K 8/64* (2006.01)
*A61K 6/75* (2020.01)

(52) Cooperative Patent Classification (CPC):
(C-Sets available)
**A61P 1/02; A61K 6/75; A61K 8/0241; A61K 8/24;
A61K 8/64; A61K 33/02; A61K 33/42; A61Q 11/00**
(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Credentis AG
5210 Windisch (CH)**

(72) Inventors:
• **Hug, Michael
  4800 Zofingen (CH)**
• **Lysek, Dominikus Amadeus
  5210 Windisch (CH)**

(74) Representative: **Moré, Solveig Helga et al
Kroher Strobel
Rechts- und Patentanwälte PartmbB
Bavariaring 20
80336 München (DE)**

(54) **ACCELERATING TOOTH REMINERALISATION AND BONE REGENERATION WITH
SELF-ASSEMBLING PEPTIDES AND AMORPHOUS CALCIUM PHOSPHATE**

(57)     The present invention relates to the field of medicinal tissue mineralisation, in particular, i.e. tooth remineralisation and bone regeneration with self-assembling peptides. Use of self-assembling peptides, such as P11-4, also designated Oligopeptide-104, in these processes leads to generation of hydroxyapatite, which is also present in natural enamel, dentin and bone. The inventors have discovered that both tooth remineralisation and bone regeneration can be significantly accelerated by adding amorphous calcium phosphate or calcium and phosphate ions that, when mixed in solution, can lead to immediate precipitation of calcium phosphate, preferably, amorphous, i.e., non-crystalline calcium phosphate. The presence of self-assembling peptide however changes the structure of the precipitated calcium phosphate, and advantageously induces crystallisation and a synergistic accelerated formation of crystalline calcium phosphate, in particular, hydroxyapatite (HA). The invention thus provides a kit comprising, a self-assembling peptide and either calcium and phosphate ions in separate compositions suitable for immediately forming calcium phosphate precipitates, e.g., amorphous calcium phosphate (ACP), if the compositions are mixed in the presence of water, or calcium phosphate particles, preferably, in the form of a suspension of calcium phosphate particles comprising at least 50% ACP. The invention also provides medical use of said kit, in particular, for in the tooth for remineralisation of lesions, mineralisation of pits and fissures, pulp capping, and for bone regeneration.

Fig. 2
G) beta a)    b)
10 µm    2 µm
G)c)

H) gamma-1 a)    b)
20 µm    2 µm
H)c)

**(Cont. next page)**

(52) Cooperative Patent Classification (CPC): (Cont.)

C-Sets
**A61K 33/02;**
**A61K 33/42**

SEQUENCE LISTING

<110> Credentis AG

<120> Accelerating tooth remineralisation and bone regeneration with self-assembling peptides and amorphous calcium phosphate

<130> 12845 P 6575 EP

<160> 3

<170> BiSSAP 1.3.6

<210> 1
<211> 11
<212> PRT
<213> Artificial Sequence

<220>
<223> P11-4

<400> 1
Gln Gln Arg Phe Glu Trp Glu Phe Glu Gln Gln
1               5                   10

<210> 2
<211> 11
<212> PRT
<213> Artificial Sequence

<220>
<223> P11-2

<400> 2
Gln Gln Arg Phe Gln Trp Gln Phe Glu Gln Gln
1               5                   10

<210> 3
<211> 11
<212> PRT
<213> Artificial Sequence

<220>
<223> P11-29

<400> 3
Gln Gln Glu Phe Glu Trp Glu Phe Glu Gln Gln
1               5                   10

**Description**

The present invention relates to the field of medicinal tissue mineralisation, in particular, i.e. tooth remineralisation and bone regeneration with self-assembling peptides. Use of self-assembling peptides, such as P11-4, also designated Oligopeptide-104, in these processes leads to generation of hydroxyapatite, which is also present in natural enamel, dentin and bone. The inventors have discovered that both tooth remineralisation and bone regeneration can be significantly accelerated by adding amorphous calcium phosphate or calcium and phosphate ions that, when mixed in solution, can lead to immediate precipitation of calcium phosphate, preferably, amorphous, i.e., non-crystalline calcium phosphate. The presence of self-assembling peptide however changes the structure of the precipitated calcium phosphate and advantageously induces crystallisation and a synergistic accelerated formation of crystalline calcium phosphate, in particular, hydroxyapatite (HA). The invention thus provides a kit comprising, a self-assembling peptide and either calcium and phosphate ions in separate compositions suitable for immediately forming calcium phosphate precipitates, e.g., amorphous calcium phosphate (ACP), if the compositions are mixed in the presence of water, or calcium phosphate particles, preferably, in the form of a suspension of calcium phosphate particles comprising at least 50% ACP. The invention also provides medical use of said kit, in particular, in the tooth, for remineralisation of lesions, mineralisation of pits and fissures, treatment of sensitive teeth, pulp capping, and for bone regeneration.

Tooth decay, also known as dental caries, is one of the most ubiquitous diseases in the world caused by acid secreting bacteria . It is a breakdown of tooth material due to bacterial metabolites, mainly acids made by bacteria when they break down food debris or sugar on the surface or in the oral biofilm. This leads to an imbalance between demineralisation and remineralisation processes. Hard tooth structures, i.e., enamel, dentin and cementum, are damaged by ongoing demineralisation, which results in carious lesions and eventually in the appearance of dental cavities. The earliest sign of a new carious lesion is the appearance of a chalky white spot on the surface of the tooth, a so-called white spot lesion (also designated an incipient carious lesion), i.e. a subsurface lesion. As the demineralisation progresses, the mineralised surface of the lesion (partially) collapses and breaks and a microcavity or a cavity, a hole in the tooth, appears. This is referred to as a (partially) cavitated carious lesion or cavity.

The classical treatment for tooth lesions, in particular, carious tooth lesions, is drilling and filling the tooth. Once a carious lesion has cavitated and a hole in the tooth appears, the common treatment applied to date is invasive. The decayed material is usually removed by using, for example, a dental handpiece ("drill"). Alternatively, a laser, a dental spoon or a chemo-mechanical system may be used to remove dental caries.

After removal (i.e. excavation) of the carious enamel and/or dentin, the missing tooth structure requires a dental restoration using dental restorative materials such as sealants, amalgam, dental composites, porcelain or gold.

However, it would be advantageous to be able to restore the natural tooth matter, which is based on hydroxyapatite, instead of losing precious hard tissue followed by filling excavated lesions with a substance foreign to the human body. Such seamless remineralisation could, e.g., reduce the incidence of secondary caries that often appears at the filling margins, or when a filling fails.

Non-invasive approaches based on remineralisation have been suggested for treatment of non-cavitated carious lesions, i.e. subsurface lesions. For example, classically, remineralisation is attempted by the application of topical fluoride.

Furthermore, enamel matrix derivatives or self-assembling peptides have been shown to be effective in the remineralisation of subsurface carious lesions (Ruan et al., 2013. Acta Biomater.9(7):7289-97; Ruan et al., 2014. J Vis Exp. 10(89), doi 10.379151606; Schmidlin et al., 2016, J Appl Oral Sci. 24(1), 31-36; Alkilzy et al., 2018, Adv Dent Res. 29(1), 42-47; Brunton et al., 2013, Br Dent J, 215:E6; Kind et al., 2017, J Dent Res., 96(7), 790-797; Kirkham et al., 2007, J Dent Res., 86, 426-430). In WO 2014/027012 A1, a method for the targeted delivery of a self-assembling peptide to a subsurface tooth lesion with the aim of remineralising carious lesions is described.

PCT/EP2020/069360 describes self-assembling peptides in the prevention and treatment of cavitated carious lesions.

In WO 2017/168183 A1, a biomimetic mineralised apatite structure based on elastin-like peptides is described for use as dental restorative material, in particular for the reconstruction of enamel, and for use in the treatment of dental diseases such as dental caries.

Furthermore, casein-based strategies have been developed for remineralisation of enamel and prevention and/or treatment of caries or tooth erosion (e.g. WO 00/06108 A1 and WO 2010/042754 A2). Casein phosphopeptide amorphous calcium phosphate complexes (CPP-ACP) and CPP-stabilised amorphous calcium fluoride phosphate complexes have been described in combination with glass-ionomer cement as remineralising dental restorative materials, e.g., in WO 02/094204 A1.

Other calcium phosphate particles, e.g., comprising amorphous calcium phosphate, have also been used for remineralisation of demineralised enamel (e.g., Weir et al., 2012, J Dent Res. 91(10):979-984; Meyer et al., 2018. Open Dent J. 12:406-423).

Amelogenin-based biomineralisation approaches for tooth repair have been described in the context of dentinal hypersensitivity, whitening or bleaching of teeth, and the treatment of caries in WO 2017/123986 A1, US 2014/0186273 A1 and US 2017/0007737 A1.

Remineralisation of demineralised enamel surfaces has also been attempted with nano-particles of carboxymethyl chitosan/amorphous calcium phosphate particles (CMC/ACP) guided by chimaeric peptides. The chimaeric peptide required for this is rather long and comprises two functional domains, comprising an amelogenin-peptide that can transform ACP into hydroxyapatite, and one peptide that has been found to specifically bind to hydroxyapatite surfaces. An enamel like structure is formed, wherein CMC/ACP nanoparticles are degraded by NaClO and guided by the chimaeric peptide into ordered and oriented arrays (Xiao et al., 2017. Dent. Mater. 33(11):1217-1228).

Self-assembling peptides such as those described in WO 2004/007532 A2 have also been shown to be very successful in remineralisation of subsurface lesions (e.g., Kirkham et al., 2007), but the problem remains that the remineralisation is not very quick, and needs several weeks to months to be come visible on dental radiographs or by change in appearance of the white spot lesion (Brunton 2013, Schlee 2017). For this reason, the self-assembling peptides alone are also not suitable for treatment of cavitated caries lesions, as, even in assembled form, i.e., as a hydrogel, they are likely to be removed from the cavity before the hydrogel becomes sufficiently remineralised and stable.

Self-assembling peptides have also been taught to be useful for regeneration of bone (e.g., WO 2004/007532 A2), with the same issue of slow mineralisation and the potential clinical drawback of lack of volume stability during said process.

Thus, in light of the state of the art, the inventors addressed the problem of overcoming at least some of these issues, advantageously, providing a composition and method for providing a rapid mineralisation of tooth lesions and defects, and/or bone regeneration. This problem is solved by the present invention as disclosed herein, in particular, in the claims.

In one embodiment, the present invention provides a kit comprising,

a) a self-assembling peptide comprising the amino acid sequence SEQ ID NO: 1 or an amino acid sequence having at least 80% identity thereto, and

b)

(i) calcium and phosphate ions in separate compositions suitable for immediately forming calcium phosphate precipitates, preferably, amorphous calcium phosphate (ACP) if the compositions are mixed in the presence of water, wherein the compositions preferably are solutions, or

(ii) calcium phosphate particles, preferably, in the form of a suspension of calcium phosphate particles comprising at least 50% ACP.

Self-assembling peptides

Preferably, the self-assembling peptide used in the present invention comprises or consists of a sequence selected from the sequences listed in Table 1 below. The self-assembling peptide that may be used in the invention have a net charge of -2 at neutral pH, which, together with their specific sequence, enables them to bind to hydroxyapatite.

**Table 1: Preferred self-assembling peptides.**

| SEQ ID NO | Peptide name | Sequence (One letter code) | % amino acid identity to P11-4 (ClustalW (2.1, standard parameters)) |
|---|---|---|---|
| SEQ ID NO: 1 | P11-4 | QQRFEWEFEQQ | 100 |
| SEQ ID NO: 2 | P11-2 | QQRFQWQFEQQ | 81.8 |
| SEQ ID NO: 3 | P11-29 | QQEFEWEFEQQ | 90.9 |

Peptides of SEQ ID NO: 1, 2 or 3 or comprising said sequences are particularly examples of self-assembling peptides of the invention. Peptides comprising the defined sequences preferably have 20 amino acids or less, e.g., 15 amino acids or less, or 12, 13 or 14 amino acids. Shorter peptides are preferred, as they are easier and cheaper to synthesize. Preferably, the self-assembling peptide comprises the sequence of SEQ ID NO: 1 or consists thereof. A peptide consisting of a sequence of SEQ ID NO: 1 is also designated P11-4 or Oligopeptide-104, and it is preferred throughout the invention.

Self-assembling peptides may be modified peptides comprising an Ac-N-terminus and/or $NH_2$-C-Terminus, preferably, both, or non-modified peptides. As non-blocked forms tend to start a deamination reaction, the termini of all self-assembling peptides, e.g., of SEQ ID NO: 1 are preferably blocked to increase stability.

The self-assembling peptides preferably do not have restriction sites for human endopeptidases. They also do not need to comprise a special recognition motif for cells.

In the kit of the invention, the self-assembling peptide may be in monomeric form, in assembled form, or a mixture thereof. In the context of the invention, the self-assembling peptide employed may undergo self-assembly at a low pH, in particular, at a pH below 7.5, e.g., P11-4. The term that the self-assembling peptides (SAPs) undergo self-assembly at a pH below 7.5 means that they are capable of self-assembly at a pH below 7.5. The ionic strength also influences the assembly state of the selected self-assembling peptide. Preferably, the self-assembling peptides used in the present invention are capable of self-assembly at a pH below 7.5 and at least physiologic ionic strength. A high ionic strength also leads to assembly of the self-assembling peptides (Maude 2011. Soft matter 7: 8085-8098, Carrick 2008. Tetrahedron 63: 7457-7467).

The skilled person will know how to determine and measure the ionic strength of a solution. The ionic strength I is generally calculated according to the formula $I = \frac{1}{2}\sum z_i^2 b_i$, wherein z is the valence factor and $b_i$ is the molality [mol/kg{$H_2O$}] of the $i^{th}$ ion concentration. The summation, $\sum$, is taken over all ions in a solution. For example, the ionic strength of a 150 mM NaCl solution is approximately 0.15 mol/L. This is also approximately the ionic strength of blood. The ionic strength of saliva present in the oral cavity, is generally much lower, such as approximately 0.04 mol/L. In the context of the invention, ionic strength in the physiological range is considered to correspond to a ionic strength of 0.15 mol/L.

If desired, the mechanical properties can be influenced by the concentration of the self-assembling peptide and additionally the type of molecules and ions present in the composition. A composition comprising a self-assembling peptide employed in the invention may e.g., comprise NaCl and/or other salts, preferably, soluble salts only, and, optionally, a biologically suitable buffer such as Tris.

In one embodiment, in the kit according to the present invention, the self-assembling peptide is in predominantly monomeric form, e.g., at least 50%, at least 70%, at least 80%, or at least 90% of the self-assembling peptides are present in a non-assembled state, e.g., in a monomeric state or in low-order aggregates. To this end, if the peptide assembles in a pH at or below 7.5, the pH of the composition may be above the pH wherein the peptide starts to undergo self-assembly (e.g., pH 7.5 for P11-4), preferably, 0.1 to 0.5 pH units above said pH, or more than 0.5 pH units above said pH. The pH may be buffered at that pH to avoid quick assembly. The inventors found that low order aggregates, apparent through the opaque colour of the solution, may be induced in the presence of calcium ions, e.g., in a concentration of 0.01 M - 0.2 M, or 0.1M-15M.

It may be beneficial if aggregation, and formation of a hydrogel starts quickly after application. Accordingly, the pH may be 0.1 to 0.5 pH units above the pH at which the peptide starts to undergo self-assembly, with or without buffering. In one embodiment, the composition may comprise dried peptide, e.g., obtainable according to WO 2014/027012. Said peptide can be dissolved in water while directly leading to a solution of monomeric peptide.

In another embodiment, the kit according to the invention comprises self-assembling peptides in predominantly assembled form, e.g., at least 50%, at least 70%, at least 80%, or at least 90% are in assembled form, or essentially assembled form, and a buffer (at a pH stabilizing the assembled form). Assembled self-assembling peptide typically forms a hydrogel.

Selection of the assembly state of the protein depends on several factors. A predominantly monomeric or low order aggregate form of the self-assembling peptide improves diffusion of the peptide into demineralized enamel, and may be particularly advantageous for treatment of subsurface lesions or small tooth lesions, e.g., partially cavitated lesions or lesions with microcavities. A predominantly assembled form may lead to quicker action of the peptide and thus improve function, in particular for cavities or for bone defects. After the self-assembling peptide and the other components of the kit are mixed (optionally, after dissolving), the pH and the ionic strength of the resulting solution will typically be decisive for assembly of the self-assembling peptide. However, the pH of the target site, e.g., a carious tooth lesion with an acidic pH, may also influence the assembly state. In particular, in an acidic carious tooth lesion, the self-assembling peptide assembles and forms a hydrogel.

(i) Calcium and phosphate ions in separate compositions

In a preferred embodiment, the kit of the present invention comprises b)(i) calcium and phosphate ions in separate compositions suitable for immediately forming calcium phosphate precipitates, preferably, amorphous calcium phosphate (ACP), if the compositions are mixed in the presence of water.

In previous attempts for rapid remineralisation of tooth lesions, if calcium phosphate was used, it was typically used in the form of calcium phosphate particles (e.g., amorphous calcium phosphate, ACP), as, e.g., taught by Xiao et al., 2017 or Tung et al. 2004 (Compendium CE2 Vol. 25 Mo. 9 (Suppl. 1)). However, ACP is instable, in particular, in acidic media, or at a pH of less than 9, and therefore it is advantageously stabilized, e.g., in the form of CMC/ACP or with casein-phosphopeptides (CPP-ACP). This also has disadvantages, e.g., the particles may need to be broken down, e.g., with NaOCl, as taught by Xiao et al.. The present invention overcomes this problem by generating calcium phosphate, e.g., ACP in situ. Calcium phosphate precipitates, typically in the form of ACP, form above a certain concentration of calcium and phosphate The calcium phosphate formed preferably is ACP, or comprises at least 50% ACP, at least initionally. Conditions for preparation of ACP are e.g., described in Comes et al., 2010. Acta Biomaterialia 6(9):3362-3378. Of course, the conditions chosen for the present invention should be biologically compatible, i.e., they should not cause damage to human tissue such as teeth, gums, bone, tendon and/or muscle tissue. Typically, that means that the solvent is biocompatible or easily degraded within the human body, i.e., typically, water. Alternatively, a mixture of water and ethanol (preferably, with up to 50% ethanol) may be used.

However, other calcium phosphates may also be formed, e.g., calcium triphosphate, brushite, pyrophosphate, octacalcium phosphate (OCP), Whitlockite or other calcium phosphate precursors for the formation of hydroxyapatite (HA). HA itself may also be directly formed, in particular, in the presence of the self-assembling peptides of the invention.

The inventors surprisingly found that the combination of self-assembling peptide of the invention and calcium and phosphate ions, that, in the absence of the self-assembling peptide, would form ACP or other non-HA crystals, shifts the balance towards formation of more ordered, crystallized forms of calcium phosphate. In particular, as shown in the examples below, enamel like or HA-like calcium phosphate crystals may be formed, which is particularly preferred, in particular, in the context of tooth treatment.

The presence of the self-assembling peptide also accelerates the formation of said calcium phosphate precipitates, so that they are immediately formed. In the context of the invention, immediately is understood to refer to a time of several seconds up to about five minutes, often within one minute. The higher the concentration, the quicker the precipitation. The time starts upon mixing, i.e., if mixing is carried out slowly, the process of precipitation also occurs more slowly.

The different calcium phosphate crystal forms can be distinguished from each other, e.g., based on their Ca/P ratio (Comes et al., 2010, Jeffre et al., 1993. Marine and Freshwater Research 44:609-634). OCP has a C/P range of about 1.33. ACP typically has a C/P ratio of about 1.35-1.45. Whitlockite is a form having a C/P range of about 1.5. HA has a C/P range of more than 1.5, in particular, 1.6 or more. HA in bone may e.g., have a C/P ration of about 1.76.

The C/P ratio can be determined, e.g., according to methods known in the art, preferably, as taught in the examples herein.

In a preferred embodiment, the composition comprising calcium ions is a solution. Alternatively, or, preferably, additionally, the composition comprising phosphate ions is a solution. The concentration of calcium and phosphate in said solutions is so high that, upon mixing, it is supersaturated, and calcium phosphate, preferably, ACP, precipitates. For use, the solutions may thus be contacted with each other, which leads to precipitation of calcium phosphate, e.g., initially, ACP.

The inventors found that suitable concentrations for a solution comprising calcium ions in a kit of the invention are, e.g., $Ca^{2+}$ in a concentration of 0.01-0.5 M. It is noted that this is the concentration in one of the compositions of the kit, i.e., upon contact and mixing with the other solution, the final concentration is lower. For example, if the solution comprising calcium ions and the solution comprising phosphate ions are mixed 1:1, wherein, preferably, the solutions are equimolar with regard to $Ca^{2+}$ and phosphate, the final concentration of calcium ions after mixing is half of that in the kit, i.e., it may be 0.005-0.25 M. However, the ratio of concentration of $Ca^{2+}$ to the concentration of phosphate can be, e.g., 1:1-2:1, in particular, 1.5:1 to 2:1, to obtain a higher Ca/P ratio. Mixing the solutions 1:1 (volume ratio) is easily possible, using, e.g., a dual chamber syringe with a mixing unit. However, to obtain a higher Ca/P ratio, if the $Ca^{2+}$ and the phosphate comprising solutions are equimolar, a higher amount of the $Ca^{2+}$ solution can also be employed, e.g., 1:1-2:1, in particular, 1.5:1 to 2:1. This shifts the balance of calcium phosphate formation towards formation of HA. Alternatively, the concentrations of the individual ions in the solution of the kit can be changed to obtain the desired stoichiometric ratio while keeping the mixing ratio 1:1.

Preferably, the kit of the invention comprises a solution comprising calcium ions in a concentration of 0.08-0.28 M. Particularly good results have been obtained using a solution comprising calcium ions in a concentration of 0.1-0.15 M, e.g., 0.14-0.15 M (e.g., with an about equimolar phosphate solution.

It is noted that the saturation or supersaturation of solutions is strongly pH-dependent. The above values are particularly suitable if the solution formed by mixing the two solutions has a neutral or physiologic pH (e.g., pH 7-7.4). Optionally, the solution comprising calcium ions has a pH of 6-7. Alternatively, it may be suitable to be dissolved in water to obtain a solution having a pH of 6-7. The solution may be buffered.

The composition comprising calcium ions comprises a calcium salt that is soluble in water, e.g., $CaCl_2$, $Ca(NO_3)_2$, $CaCO_3$, or a mixture thereof. The salt may be dissolved or suitable for being dissolved in a solution. In a preferred embodiment, the composition comprises $CaCl_2$.

The composition comprising calcium ions, e.g., the solution, may further comprise $Mg^{2+}$ and or $Sr^{2+}$ ions. The

ratio of $Ca^{2+}$ to $Mg^{2+}$ and/or $Sr^{2+}$ may be, e.g., about 0.3.

As explained before, the composition comprising phosphate may be used in about equimolar concentration. Alternatively, more Ca ions than phosphate ions may be used.

For example, the kit of the invention may comprise a solution comprising phosphate ions in a concentration of 0.01-0.5 M. Preferably, the solution comprises phosphate ions in a concentration of 0.08-0.28 M. Particularly good results have been obtained with a solution comprising phosphate ions in a concentration of 0.1-0.15 M, e.g., 0.14-0.15 M.

The inventors found that different concentrations of calcium and phosphate ions, respectively, e.g., in equimolar amounts, led to formation of different calcium phosphates in combination with self-assembling peptides. However, even though, e.g., the remineralisation solution of Kirkham et al., which is intended to mirror saliva, is a supersaturated calcium phosphate solution, no immediate calcium phosphate precipitate or crystal forms (cf. sample beta (without peptide) and delta (with peptide). The concentration of calcium and phosphate ions in the kit thus has to be higher, e.g., at least 0.01 M, respectively, optionally, at least 0.05 M, respectively. The inventors found an optimal concentration of calcium and phosphate ions (in the unmixed solutions of the kit of the invention) of about 0.1-0.15 M. Under these conditions, calcium phosphate precipitated, which can then, if still required, recrystallize to form HA, e.g., in a tooth or bone lesion.

If the concentration of calcium and phosphate ions is very high (e.g., in samples II CaNa or II CaPaNa), calcium phosphate immediately crystallizes, but the structure thereof is less ordered and does not appear to be significantly influenced by the self-assembling peptide. In the presence of the self-assembling peptide, recrystallization to form HA is still expected, but concentrations of calcium and phosphate ions below 0.3 M or at most 0.28 M are preferred in the context of the invention.

The composition comprising phosphate ions, i.e., the solution, may comprise a phosphate salt that is dissolved or suitable for being dissolved in the solution. The salt may e.g., be $Na_2HPO_4$, $NaH_2PO_4$, $Na_3PO_4$, $K_2HPO_4$, $KH_2PO_4$, $K_3PO_4$ or a mixture thereof. $Na_3PO_4$ is very basic, while $NaH_2PO_4$ is rather acidic (about pH 5), and $Na_2HPO_4$ leads to a pH of about 8. In a preferred embodiment, the solution comprising phosphate ions comprises $Na_2HPO_4$, e.g., as the only phosphate salt .The solution comprising phosphate ions may e.g., have a pH of 6-10, preferably, of 7-9, optionally, of 7.4-8. At this pH, the self-assembling peptides, e.g., of SEQ ID NO: 1, are monomeric. The pH can also be modified by addition of other buffers, or buffered by use of a mixture of different phosphate salts, e.g., $Na_2HPO_4$ and $NaH_2PO_4$.

The composition comprising calcium ions may also be a dry composition suitable for dissolving in water to provide a solution comprising calcium ions. The dry composition optionally is lyophilised, air-dried, vacuum dried or spray-dried.

Similarly, the composition comprising phosphate ions may be a dry composition suitable for dissolving in water to provide a solution comprising phosphate ions. The dry composition optionally is lyophilised, air-dried, vacuum dried or spray-dried.

It is however noted that it is not required that the composition is or the compositions are a solution, or that the composition is/are dissolved prior to administration. For example, the composition can be a dry composition such as a chewing gum comprising at least one of the ions ($Ca^{2+}$ or phosphate) or both, in different parts thereof, and/or the self-assembling peptide. A solution can then be formed e.g., with the saliva while the chewing gum is chewed. The inventors have found that this also leads to precipitation of calcium phosphate. For assessing the required amount of ions, the concentration should be adjusted to the saliva formed in the mouth, e.g., saliva flow is ~1-5 mL/min.

(ii) Calcium phosphate particles

In another embodiment, the kit of the present invention comprises b) calcium phosphate particles. The calcium phosphate particles and the self-assembling peptide are comprised in the kit separately from each other, preferably, in separate compositions, e.g., separate containers, or at least in separate parts of a composition (e.g., in a striped toothpaste).

The calcium phosphate particles are not part of a dental sealant, in particular, not part of a dental cement such as a glass-ionomer cement. The composition comprising the calcium phosphate particles of the kit of the invention is not a composition suitable or intended for polymerisation of non-peptide polymers, i.e., comprising monomers of such polymers or polymers thereof. Preferably, the composition also does not comprise an organic solvent.

Preferably, the composition comprising the calcium phosphate particles of the kit of the invention is a suspension of calcium phosphate particles in water. Of course, depending on solubility, temperature etc., some of the calcium phosphate in the suspension may be dissolved, but the majority of the calcium phosphate is not dissolved and in particulate form.

The composition comprising the calcium phosphate particles of the kit of the invention may also be a composition suitable for preparing a suspension of calcium phosphate particles in water by adding water and, optionally, mixing. A suspension of calcium phosphate particles in saliva, e.g., in the mouth of a subject, may also be formed if the particles are suspended in saliva. Such compositions suitable for forming suspensions may e.g., be dry, e.g., water-free compositions, such as a powder. They may also be incorporated in or bound to a support, e.g., a chewing gum, lozenge etc.,

from which they can be set free upon chewing or sucking, thus forming a suspension in saliva.

The composition can also be or comprise a foamed composition of calcium phosphate, as such compositions have a high surface that allows good access for the solvent (e.g., water). For example, mechanical stress such as chewing may set calcium phosphate particles free from such compositions.

In the suspension, the particles should be free to form complexes with the self-assembling peptides and/or to adhere to hydroxyapatite material to which they are contacted, e.g., tooth or bone.

The calcium phosphate particles in the kit of the invention may be any form of calcium phosphate particles, e.g., in different crystalline and non-crystalline forms.

Calcium phosphate particles may, in the context of the present invention, be monocalciumphosphat-monohydrate (MCPM) $Ca(H_2PO4)_2*H_2O$, monocalciumphosphate anhydrate (MCPA) $Ca(H_2PO4)_2$, dicalciumphosphate dihydrate (DCPD, Brushit), $CaHPO_4*2H_2O$, dicalciumphosphate anhydrate (DCPA, Monetit) $CaHPO_4$], Octacalciumphosphate (OCP) $Ca_8(HPO_4)_2(PO_4)_4 * 5H_2O$, $\alpha$-tricalciumphosphate (a-TCP) $\alpha$-$Ca_3(PO_4)_2$, $\beta$-tricaidumphosphate ($\beta$-TCP) $\beta$ - $Ca_3(PO_4)_2$, amorphous calcium phosphate (ACP) $Ca_x(PO_4)_y * nH_2O$, calcium-deficient hydroxyapatite (CDHA) $Ca_{10}$-x$(HPO_4)_x(PO_4)_6$-x$(OH)_{2-x}$ $(0<x<1)$, hydroxyapatite (HA) $Ca_{10}(PO_4)_6(OH)_2$, or tetracalciumphosphate (TTCP) $Ca_4(PO_4)_2O$), or a mixture of different calcium phosphates. In one embodiment, the particles or have a degree of crystallinity of 50% or less, e.g., 40% or less, 30% or less, 20% or less or 10% or less, or consist of amorphous, non-crystalline calcium phosphate.

Preferably, they comprise ACP, e.g., at least 50% ACP. The calcium phosphate particles may also comprise at least 80% ACP. Optionally, they may essentially consist of ACP. The inventors found that this is advantageous, because the ACP is not very stable and can recrystallize in more ordered HA crystals in combination with ordered structures of self-assembling peptides. Optionally, the calcium phosphate particles are in the form of a suspension of calcium phosphate particles comprising at least 50% ACP, e.g., at least 80% ACP.

Dry, in particular, water-free compositions comprising calcium phosphate particles in the form of ACP, e.g, as a powder, have the advantage that the ACP is stable for a longer time. The ACP may also be a stabilized form of ACP, e.g, stabilized with casein-phosphopeptides, i.e., CPP-ACP. Alternatively, the ACP may be CMC/ACP.

The calcium phosphate particles may also or alternatively comprise OCP, optionally at least 50% OCP. The calcium phosphate particles may also or alternatively comprise tricalcium phosphate (TCP), optionally at least 50% TCP. The TCP may be beta-TCP.

In one embodiment, the calcium phosphate particles comprise HA, optionally, at least 50% HA. they may also consist of HA particles. In this case, no recrystallisation of the calcium phosphate is required. That can be advantageous in some cases, e.g., in bone providing volume stability. Hydroxyapatite may be substituted hydroxyapatite e.g., carbonate hydroxyapatite and zinc carbonate hydroxyapatite, or pure calcium phosphate, preferably, in crystalline form. In the context of the invention, reference to calcium phosphate or hydroxyapatite includes reference to derivatised calcium phosphates or hydroxyapatites of this kind unless otherwise mentioned. Of course, the calcium phosphate or hydroxyapatite may also consist of $CaPO_4$ (and of course crystal water as appropriate for the respective crystal form) only.

Hydroxyapatite particles may e.g., be obtained according to methods disclosed, e.g., in Roveri, Battistelli et al., 2009. J. Nanomater. Nanocomposites for Engineering Applications 1-9, EP 1 762 215 A1, US 20050037948 A1, US 20080075675 A1, US 20100247589 A1, US 20100297203 A1, WO 2007/137606 A1, or WO 2013/068020 A1. Preferably, the hydroxyapatite is obtainable according to WO 2007/137606 A1 and can be commercially obtained from Budenheim, Budenheim, Germany or Omya International AG, Switzerland.

Optionally, at least 50% of said calcium phosphate particles may have a size of 20 nm to 50 mm, e.g., 1 $\mu$m to 5 mm or, 20 nm to less than 1 $\mu$m. Preferably, at least 80%, at least 90% of particles, at least 95% or 100% of the particles have the respective size. The size of the mineral particles is typically measured by granulometry, e.g., with a light scattering particle size distribution analyzer (such as LA-950, Horiba, Kyoto, Japan). The form of the crystals preferably is needle-shaped, but it may also be rod-shaped or acicular.

Different sizes of the calcium phosphate particles, e.g., the ACP particles, are advantageous for different purposes. For example, for bone regeneration, large particles of $\mu$m or mm size (e.g., 1 $\mu$m to 50 mm or 500 $\mu$m to 5 mm) can be advantageous, because they add extra stability to the site where bone regeneration is needed. In contrast, for remineralization of teeth, a homogenous formation of substitute enamel or dentin is a high interest. Thus, in this context, the size of the particles should be lower, e.g., in the nanometer range (e.g., 20nm to less than 1 $\mu$m, optionally, 50 nm-500 nm).

In another embodiment, the calcium phosphate particles do not comprise HA. Other calcium phosphates recrystallize, and thus form new, seamless structures with the self-assembling peptides more easily. Bone remodelling can also occur more easily with other calcium phosphates.

## The composition comprising the self-assembling peptide

The kit of the invention comprises is not a single homogenous composition, but comprises at least two distinct compositions, optionally, three or more compositions. The kit of the invention comprising, separately, calcium and phosphate ions, may comprise,

a) in a first composition, the self-assembling peptide, and
b) in a second composition, the phosphate ions, and,
c) in a third composition, the calcium ions.

It is thus possible, but not required that all components of the kit are in three distinct compositions. Instead, the self-assembling peptide can also be part of one of said compositions together with one of said ions. This may facilitate administration and may have further advantages, as described below.

In one embodiment, the composition comprising the self-assembling peptide may also comprise the phosphate ions, e.g., the self-assembling peptide may be part of the composition, e.g., solution comprising phosphate ions, or the dry powder comprising phosphate ions that is suitable for forming a solution thereof.

Alternatively, and preferably, the composition comprising the self-assembling peptide further comprises the calcium ions, e.g., the self-assembling peptide is part of the composition (e.g., solution) comprising calcium ions, or the dry powder comprising calcium ions that is suitable for forming a solution thereof. As shown in the examples below, it surprisingly makes a difference in which composition the self-assembling peptide is incorporated. Under otherwise identical conditions, in sample gamma-2, wherein the self-assembling peptide was incorporated in the solution comprising calcium ions, the Ca/P ratio in the precipitate formed was about 1.72, i.e., it was much higher than in the sample gamma-1. Thus, if the self-assembling peptide is incorporated in the composition comprising the calcium ions, a HA precipitate directly forms. In the other sample, the precipitate can later recrystallize to form HA, but HA is not directly formed.

Without intending to be bound by the theory, it is believed that the self-assembling peptide and the calcium ions may already form complexes before the phosphate is added, thus facilitation nucleation of regular HA crystals templated by the structure of the self-assembling peptide (Thomson 2014. Caries Res (48):411). This is supported by the appearance of the solution comprising calcium ions and self-assembling peptide, which is opaque instead of clear.

If the self-assembling peptide is contained in a solution, it may be present in a concentration e.g., of 0.01-40 mg/mL. Particularly good results have been found in a composition comprising the self-assembling peptide in a concentration of 0.1-30 mg/mL, optionally, 1-25 mg/mL.

The self-assembling peptide may also be contained in the solution in a concentration of 10-20 mg/mL, optionally, 15-20 mg/mL. Of note, this concentration is also defined with regard to the solution contained in the kit, i.e., before mixing with the other ion-containing solution. The concentration in the solution formed after the components of the kit are contacted with each other may e.g., be 0.005-20 mg/mL, 0.05-15 mg/mL or 0.5-12.5 mg/mL.

Fluoride

The kit of the invention may further comprise fluoride ions, wherein the fluoride ions are in solution or in the form of a soluble fluoride salt. Advantageously, fluoride ions were shown to lead to direct formation of HA crystals under conditions that in the absence of fluoride would lead to formation of calcium phosphate having a lower Ca/P ratio. In the sample CaPaNaF, fluoride ions (in the form of NaF) where incorporated in the solution comprising phosphate and self-assembling peptide, and led to direct formation of calcium phosphate having a high Ca/P ratio or more than 2, i.e., HA.

A soluble fluoride salt may be, e.g., $NaF$, $NH_3F$, $MgF_2$, $SrF_2$, $Na_2PFO_3$, $SnF$ or a mixture thereof, optionally, NaF. NaF or $NH_3F$ are routinely used in dental care products such as toothpastes.

The fluoride ions may be in a separate composition, or they may be comprised in the composition comprising phosphate ions, wherein said composition optionally further comprises the self-assembling peptide. Alternatively, they may be comprised in the composition comprising calcium ions, wherein said composition optionally further comprises the self-assembling peptide.

As shown in the examples below, it surprisingly makes a difference in which composition the self-assembling peptide is incorporated. Under otherwise identical conditions, in sample gamma-2, wherein the self-assembling peptide was incorporated in the solution comprising calcium ions, the Ca/P ratio in the precipitate formed was about 1.72, i.e., it was much higher than in the sample gamma-1. Thus, if the self-assembling peptide is incorporated in the composition comprising the calcium ions, a HA precipitate directly forms. In the other sample, the precipitate can later recrystallize to form HA, but HA is not directly formed.

Forms of the compositions

Each of the compositions of the kit of the invention may be a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy. For example, the self-assembling peptide may be provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy. The calcium ions may be provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy. The phosphate ions may be provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy. Alternatively, the calcium phosphate (in particle form) may be provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy. Those forms intended to be chewed or sucked, e.g, chewing-gum, toffee, lozenge, candy, or also toothpaste, a tooth gel, a mouthwash, a mouth spray, an oral care foam are for use on tooth treatment, while, e.g., putty is typically used for bone treatment or pulp capping. Other forms can be used for both applications.

As stated, preferred compositions of the invention are solutions, e.g., in water. Alternative preferred compositions of the invention are dry, e.g., water-free powders that are suitable for being dissolved or, in the case of the calcium phosphate particles, resuspended in water.

If two compositions of the kit of the invention are provided that are both solutions or dispersions (e.g., one comprising the self-assembling peptide and the other calcium phosphate particles, or, preferably, a solution comprising calcium ions and a solution comprising phosphate ions, one of them further comprising the self-assembling peptide, as described above) the invention also provides a dual chamber syringe comprising, in each chamber, one of the compositions of the kit of the invention. A mixing unit may be affixed to the dual chamber syringe. A dual chamber syringe is particularly advantageous for targeted treatment, e.g., treatment of a tooth lesion or a bone lesion. Alternatively, solutions or suspensions can be mixed before administration, and then administered to a tooth or bone lesion.

The invention also provides a syringe comprising the solution comprising phosphate ions and a dry composition in another container comprising calcium ions, wherein the self-assembling peptide preferably is part of the composition comprising calcium ions, so that the solution can be used to dissolve the dry composition, and then, to apply the mixed solution.

Syringes, single or multi-chamber, can be advantageously used for targeted treatment, e.g., for targeting a tooth lesion such as a subsurface lesion. Bone lesions can also be targeted.

Alternatively, solutions can also be used, e.g., as a mouthwash, or oral care foam, e.g., for increasing tooth remineralisation. This can, e.g., be of interest for treatment of sensitive teeth or of tooth erosion.

The kit of the invention may also be a solid or semi-solid composition. Such solid or semi-solid compositions may e.g. comprise

a) in a first part of the composition, the self-assembling peptide and the calcium ions, and
b) in a second part of the composition, the phosphate ions.

Alternatively, they may comprise

a) in a first part of the composition, the self-assembling peptide and the phosphate ions, and
b) in a second part of the composition, the calcium ions, or

a) in a first part of the composition, the self-assembling peptide, and

b) in a second part of the composition, the phosphate ions, and,

c) in a third part of the composition, the calcium ions, or

a) in a first part of the composition, the self-assembling peptide, and

b) in a second part of the composition, the calcium phosphate particles.

Examples of semi-solid or solid kits are granules, candies, tablets, toffees, mints or lozenges with different layers or chewing gums. Both layers may be equally accessible to saliva upon chewing or sucking, or exposure to body fluids such as blood or buffer/water after administration into a bone lesion, or one of the parts of layers may surround the other.

For example, in these solid or semi-solid kits comprising different parts, the first part of the composition may surround the second part. As the first part comprises the self-assembling peptide, the self-assembling peptide is thus first administered to the subject, and e.g., the tooth contacted with it. Consequently, and without intending to be bound by the theory, the self-assembling peptide may bind to the HA structures of the tooth, and mediate ordered binding of the calcium phosphate that forms later to the tooth. Of course, the order of administration may also be chosen in this way if solutions are administered to a tooth or bone. It is believed this is less important for bone regeneration, as here, the volume is typically much larger, and good mixing or simultaneous administration may be more important.

Thus, alternatively, the second part of the composition may surround the first part.

Water-free solid or semi-solid compositions may be particularly useful if it is of interest that the self-assembling peptide remains monomeric through storage.

In one embodiment, the semi-solid kit is a striped toothpaste. Stripe toothpaste and methods of maintaining different compositions in a toothpaste (or tooth gel) substantially separate are well known in the art. Of course, the colours of the different compositions in striped toothpaste can be the same or different. A toothpaste of the invention may comprise further components that are typically used in toothpastes, e.g., surfactants, cleaning agents, active ingredients, flavors, sweeteners.

If the kit is a semi-solid or solid composition with different parts, the first and the second part of the composition are typically suitable for being mixed if the composition is dissolved or dispersed in a water-based solution, e.g., water. Of course, for administration to a tooth, the composition may alternatively be dispersed or dissolved in saliva. For administration to a bone, the composition may e.g., applied dry or hydrated/ dispersed or dissolved in a body fluid such as blood, in water, saline or other water-based buffer.

Medical use

The kit of the present invention may be used for research purposes, but preferably, it is for use in medicine.

Mainly, it is for use in inducing tooth remineralization or bone regeneration, preferably, in a subject in need thereof. The subject typically is a human subject. Advantageously, said remineralisation or regeneration is accelerated compared the absence of said calcium and phosphate ions.

In one embodiment, the kit of the invention is for use in inducing mineralization or remineralization in or on a tooth of a subject, preferably, for treating a tooth lesion or cavity. The tooth lesion may be a caries (or carious) lesion. It may also be broken enamel or dentin, typically, broken enamel, or cavitated enamel or dentin. The kit of the invention may also be for use in filling pits and/or fissures, both in the presence or absence of lesions in said pits and fissures. A further use is for the treatment of tooth sensitivity, wherein dentinal tubuli may be occluded or blocked by a protective barrier made of the invention.

In one embodiment, the tooth lesion is a subsurface lesion. In this case, the self-assembling peptide optionally is monomeric, e.g., in dry, for example, lyophilized or air-dried or spray-dried form together with a buffer to maintain the peptide in monomeric state after dissolving in water, as known in the art and referenced herein. The kit preferably comprises calcium and phosphate ions, separately, because small, nucleating crystals may enter into subsurface lesions more easily than calcium particles. The composition comprising the self-assembling peptide (and, optionally, calcium or phosphate ions (preferably, calcium ions) may, e.g., be administered first, and the composition comprising the other ion second, shortly after the first (preferably, within at most 10, preferably, at most 5 minutes). Mixing, e.g., with a dual chamber syringe and simultaneous administration is however also possible.

In one embodiment, the tooth lesion is a cavitated lesion. In this embodiment, the practically instantaneous stability provided by the precipitated calcium phosphate or the calcium phosphate particles is particularly important. With self-assembling peptide alone, even though a hydrogel forms, it is typically washed away from large cavitated lesions before remineralisation. The acceleration of remineralisation provided by the present invention is thus of great benefit. To further stabilize the structure comprising self-assembling peptide and calcium phosphate, preferably, HA formed, UV treatment may be carried out to cross-link peptides. The kit may comprise calcium and phosphate separately, or, preferably, calcium phosphate particles, which advantageously have a size of 20 nm-5000 nm, optionally, 100-1000 nm.

Further, the kit of the invention, or the mixed compositions thereof, may be for use in filling the bottom of a cavity, with another dental sealant covering it as a top layer that is in contact with the saliva, e.g., a dental cement such as a glass-ionomer cement, or a dental sealant comprising components which are capable of polymerizing, such as acidic polymer selected from the group comprising an acrylate and methacrylate, ionomer, giomer, ormocer® and any other suitable polymer and/or a monomeric form thereof. Protected by the top layer, the combination of self-assembling peptide and calcium phosphate may form a remineralized substitute dental material that is very similar to enamel and/or dentin.

In one embodiment, the kit of the present invention is for use in pulp capping. Here, the material formed by combining the components of the kit of the invention can protect the pulp from the often toxic effect of the dental sealant used above it, by placing a protective biocompatible layer of calcium phosphate over the dental pulp.

In one embodiment, the kit of the invention thus further comprises, separately from the other components, a dental sealant such as a glass-ionomer cement, or a dental sealant comprising components which are capable of polymerizing, such as acidic polymer selected from the group comprising an acrylate and methacrylate, ionomer, giomer, ormocer® and any other suitable polymer and/or a monomeric form thereof.

The kit of the invention may also be for use in treatment or prevention of caries, wherein the treated region comprises a plurality, preferably, all teeth of a subject regardless of a diagnosis of active caries. Compositions such as a mouthwash, a toothpaste, or the other solid or semi-solid compositions defined above are particularly suitable for this use.

The kit of the invention may also be for use in treating tooth sensitivity. Here, it is not important that the components of the kit can enter into a subsurface lesion. Rather, they are intended to form a protective layer on a tooth or form precipitates within the dentinal tubuli. This can be particularly helpful, e.g., in case of tooth sensitivity, but it can also serve to protect the teeth from future acidic challenges, and thus prevent caries or dental erosion. Prevention refers to reduction of the incidence. In this embodiment, it is thus preferred if the self-assembling peptide is provided in assembled form, or if the compositions are suitable for inducing assembly of the self-assembling peptide when mixed. The kit may comprise calcium and phosphate separately, or calcium phosphate particles, which preferably have a size of 20 nm-5000 nm, optionally, 100-1000 nm.

In general, if calcium phosphate particles are employed in the context of tooth remineralisation, they typically have a size of 20 nm-5000 nm, optionally, 100-1000 nm to enable formation of a homogenous matrix.

In another embodiment, the kit of the invention is for use in inducing regeneration in a bone of a subject, in particular, in a bone lesion. The combination of the invention is able to provide the bone, in particular, the lesion with immediate increased stability compared to administration of self-assembling peptide alone. This is of high importance for treatment of bone lesions. The self-assembling peptides further guide the formation of HA from the calcium phosphate applied. They also provide a template for cell migration.

The bone defect or bone lesion may e.g., be caused by a tumor or by trauma. The kit may also be for use in augmentation or reconstructive treatment of the alveolar ridge, for filling of periodontal defect, or for filling of a defect after root resection, apicoectomy, cystectomy, for filling of an extraction socket to enhance preservation of the alveolar ridge, for elevation of the maxillary sinus floor, for filling a periodontal defect or a peri-implant defect. It can also be applied in orthopaedic indications such as joint implants (e.g. hip-implants) or spinal fusion.

For filling of a periodontal defect, the kit of the invention may be for use in conjunction with a product intended for Guided Tissue, Regeneration (GTR) and Guided Bone Regeneration (GBR). For use in filling of a peri-implant defect, it is preferably used in conjunction with a product intended for Guided Bone Regeneration (GBR).

In the context of bone regeneration, the kit preferably comprises calcium phosphate particles having a size of 1 $\mu$m-50mm, optionally, 100-5000 $\mu$m or 1-2 mm. Such calcium phosphate particles provide the bone lesion with particular stability. Further, in this context, an assembled form of the self-assembling peptide is preferably used.

The invention also provides a method of treatment, comprising administering the components of the kit of the invention, as described herein, to a tooth or bone of a subject in need thereof, typically, to a tooth or bone lesion. Optionally, the method comprises first administering the composition comprising the self-assembling peptide first. Alternatively, the composition comprising the self-assembling peptide is administered second. Alternatively, the components of the kit are administered essentially simultaneously, which is typically the case if the lesions are of a comparatively great dimension, e.g., for filling a cavitated lesion, for pulp capping or for treatment of a bone defect.

In summary, the kit and method of the invention advantageously accelerate tooth remineralization or bone regeneration, e.g., compared to administration of either self-.assembling peptide or calcium phosphate alone, and can thus be used to this end.

The invention is further exemplified by the following embodiments, examples and figures, which are meant to illustrate, but not to limit the invention. All references cited herein are herewith fully incorporated.

In the context of the invention, "a" is intended to encompass the plural, i.e., "a tooth" also refers to a plurality of teeth, e.g., all teeth of a subject. About means +/- 10%.

Embodiments

The invention provides, e.g., the following embodiments;

1. A kit comprising,

a) a self-assembling peptide comprising the amino acid sequence SEQ ID NO: 1 or an amino acid sequence having at least 80% identity thereto, and

b)

(i) calcium and phosphate ions in separate compositions suitable for immediately forming calcium phosphate precipitates if the compositions are mixed in the presence of water, wherein the compositions preferably are solutions, or

(ii) calcium phosphate particles, preferably, in the form of a suspension of calcium phosphate particles comprising at least 50% ACP.

2. The kit of embodiment 1, comprising b) calcium and phosphate ions in separate compositions suitable for immediately forming calcium phosphate precipitates, preferably, forming amorphous calcium phosphate (ACP), if the compositions are mixed in the presence of water.

3. The kit of any of embodiments 1 or 2, wherein the composition comprising calcium ions is a solution.

4. The kit of any of embodiments 1-3, wherein the composition comprising phosphate ions is a solution.

5. The kit of any of embodiments 1-4, wherein the composition comprising calcium ions is a dry composition suitable for dissolving in water to provide a solution comprising calcium ions, wherein the dry composition optionally is lyophilised, air-dried or spray-dried.

6. The kit of any of embodiments 1-5, wherein the composition comprising phosphate ions is a dry composition suitable for dissolving in water to provide a solution comprising phosphate ions, wherein the dry composition optionally is lyophilised, air-dried or spray-dried.

7. The kit of any of embodiments 1-6, wherein the solution is a water-based solution, wherein the solvent of the solution preferably is water.

8. The kit of any of embodiments 1-7, wherein the solution comprises ethanol as solvent.

9. The kit of any of embodiments 1-8, wherein the solvent of the solution is a mixture of water and ethanol as solvent.

10. The kit of any of embodiments 1-9, wherein the solution comprising calcium ions comprises $Ca^{2+}$ in a concentration of 0.01-0.5 M.

11. The kit of any of embodiments 1-10, wherein the solution comprising calcium ions comprises $Ca^{2+}$ in a concentration of 0.08-0.28 M.

12. The kit of any of embodiments 1-11, wherein the solution comprising calcium ions comprises $Ca^{2+}$ in a concentration of 0.1-0.15 M, e.g., 0.14-0.15 M.

13. The kit of any of embodiments 1-12, wherein the solution comprising calcium ions comprises a salt selected from the group consisting of $CaCl_2$, $Ca(NO_3)_2$, $CaCO_3$, or a mixture thereof, wherein the salt is dissolved or suitable for being dissolved in the solution.

14. The kit of any of embodiments 1-13, wherein the solution comprising calcium ions comprises $CaCl_2$.

15. The kit of any of embodiments 1-14, wherein the solution comprising calcium ions further comprises $Mg^{2+}$ and or $Sr^{2+}$, wherein the ratio of $Ca^{2+}$ to $Mg^{2+}$ and/or $Sr^{2+}$ is about 0.3.

16. The kit of any of embodiments 1-15, wherein the solution comprising calcium ions has a pH of 6-7.

17. The kit of any of embodiments 1-16, wherein the solution comprising phosphate ions comprises phosphate in a concentration of 0.01-0.5 M.

18. The kit of any of embodiments 1-17, wherein the solution comprising phosphate ions comprises phosphate in a concentration of 0.08-0.28 M.

19. The kit of any of embodiments 1-18, wherein the solution comprising phosphate ions comprises phosphate in a concentration of 0.1-0.15 M, e.g., 0.14-0.15 M.

20. The kit of any of embodiments 1-19, wherein the solution comprising phosphate ions comprises a salt selected from the group consisting of $Na_2HPO_4$, $NaH_2PO_4$, $Na_3PO_4$, $K_2HPO_4$, $KH_2PO_4$, $K_3PO_4$ or a mixture thereof, wherein the salt is dissolved or suitable for being dissolved in the solution.

21. The kit of any of embodiments 1-20, wherein the solution comprising phosphate ions comprises comprises $Na_2HPO_4$.

22. The kit of any of embodiments 1-21, wherein the solution comprising phosphate ions has a pH of 6-10, preferably, of 7-9, optionally, of 7.4-8.

23. The kit of any of embodiments 1-22, wherein the self-assembling peptide is a part of the composition comprising calcium ions, wherein the composition preferably is a solution.

24. The kit of any of embodiments 1-22, wherein the self-assembling peptide is a part of the composition comprising phosphate ions, wherein the composition preferably is a solution.

25. The kit of any of embodiments 1-24, wherein the self-assembling peptide is contained in the solution in a concentration of 0.01-40 mg/mL.

26. The kit of any of embodiments 1-25, wherein the self-assembling peptide is contained in the solution in a concentration of 0.1-30 mg/mL, optionally, 1-25 mg/mL.

27. The kit of any of embodiments 1-26, wherein the self-assembling peptide is contained in the solution in a concentration of 10-20 mg/mL, optionally, 15-20 mg/mL.

28. The kit of any of embodiments 1-27, further comprising fluoride ions, wherein the fluoride ions are in solution or in the form of a soluble fluoride salt.

29. The kit of embodiment 28, wherein the soluble fluoride salt is selected from the group comprising NaF, $NH_3F$, $MgF_2$, $SrF_2$, or a mixture thereof, optionally, NaF.

30. The kit of any of embodiments 1-29, wherein the composition comprising phosphate ions comprises the fluoride ions, wherein said composition optionally further comprises the self-assembling peptide.

31. The kit of any of embodiments 1-30, comprising b) calcium phosphate particles.

32. The kit of embodiment 31, wherein the calcium phosphate particles comprise ACP, preferably, at least 50% ACP.

33. The kit of any of embodiment 31-32, wherein the calcium phosphate particles comprise at least 80% ACP, optionally, essentially consist of ACP.

34. The kit of any of embodiment 32-33, wherein the ACP is CPP-ACP.

35. The kit of any of embodiments 31-34, wherein the calcium phosphate particles comprise OCP, optionally at least 50% OCP.

36. The kit of any of embodiments 31-35, wherein the calcium phosphate particles comprise OCP, optionally at least 50% OCP.

37. The kit of any of embodiments 31-36, wherein the calcium phosphate particles comprise TCP, optionally, at least 50% TCP.

38. The kit of any of embodiments 31-37, wherein the calcium phosphate particles comprise HA, optionally, at least 50% HA, e.g., consist of HA.

39. The kit of any of embodiments 31-38, wherein the calcium phosphate particles are in an aqueous suspension, optionally, an aqueous suspension of ACP.

40. The kit of any of embodiments 31-39, wherein at least 50% of said particles have a size of 20 nm to 50 mm, optionally, 20 nm to less than 1 $\mu$m.

41. The kit of any of embodiments 1-40, wherein at least 50% of said particles have a size of 1 $\mu$m to 50 mm, optionally, of 0.5-5 mm.

42. The kit of any of embodiments 1-41, wherein the self-assembling peptide comprises the amino acid sequence SEQ ID NO: 1, preferably, wherein it consists of the amino acid sequence SEQ ID NO: 1.

43. The kit of any of embodiments 1-42, wherein the self-assembling peptide is in monomeric form, in assembled form, or a mixture thereof.

44. The kit of any of embodiments 1-43, wherein at least 50% of the self-assembling peptide are in monomeric form, optionally, at least 80%, e.g., essentially all of the self-assembling peptide.

45. The kit of any of embodiments 1-43, wherein at least 50% of the self-assembling peptide are in assembled form, optionally, at least 80%, e.g., essentially all of the self-assembling peptide.

46. The kit of any of embodiments 1-45, wherein the self-assembling peptide is provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet or a candy, a putty, a powder, or granules.

47. The kit of any of embodiments 1-30 or 41-46, wherein the calcium ions are provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a candy, a putty, a powder, or granules.

48. The kit of any of embodiments 1-30 or 41-47, wherein the phosphate ions are provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a candy, a putty, a powder, or granules.

49. The kit of any of embodiments 1 or 31-46, wherein the calcium phosphate is provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a candy, a putty, a powder, or granules.

50. The kit of any of embodiments 1-30 or 41-48, comprising,

   a) in a first composition, the self-assembling peptide, and
   b) in a second composition, the phosphate ions, and,
   c) in a third composition, the calcium ions.

51. The kit of any of embodiments 1-23 or 25-30 or 41-48, that is a solid or semi-solid composition, comprising,

a) in a first part of the composition, the self-assembling peptide and the calcium ions, and
b) in a second part of the composition, the phosphate ions

52. The kit of any of embodiments 1-22 or 24-30 or 41-48, that is a solid or semi-solid composition, comprising,

a) in a first part of the composition, the self-assembling peptide and the phosphate ions, and
b) in a second part of the composition, the calcium ions.

53. The kit of any of embodiments 1-30 or 41-48, that is a solid or semi-solid composition, comprising,

a) in a first part of the composition, the self-assembling peptide, and
b) in a second part of the composition, the phosphate ions, and,
c) in a third part of the composition, the calcium ions.

54. The kit of any of embodiments 1 or 31-46 or 49 that comprises the self-assembling peptide and the calcium phosphate particles separately from each other, preferably, in separate compositions.
55. The kit of any of embodiments 1 or 31-46 or 49 or 54 wherein the calcium phosphate particles are not part of a dental sealant, in particular, not part of a dental cement.
56. The kit of any of embodiments 1 or 31-46 or 49 or 54-55, that is a solid or semi-solid composition, comprising,

a) in a first part of the composition, the self-assembling peptide, and
b) in a second part of the composition, the calcium phosphate particles.

57. The kit of any of embodiments 50-53 or 56, wherein the first part of the composition surrounds the second part.
58. The kit of any of embodiments 50-53 or 56, wherein the second part of the composition surrounds the first part.
59. The kit of any of embodiments 50-53 or 56, wherein the composition is a striped toothpaste.
60. The kit of any of embodiments 50-53 or 56, wherein the first and the second part of the composition are suitable for being mixed if the composition is dissolved or dispersed in water.
61. The kit of any of embodiments 1-60 for use in medicine.
62. The kit of any of embodiments 1-61 for use in inducing tooth remineralization or bone regeneration, preferably, in a subject in need thereof.
63. The kit of embodiment 62, wherein said remineralisation or regeneration is accelerated compared the absence of said calcium and phosphate ions.
64. The kit of any of embodiments 1-63 for use in inducing remineralization in or on a tooth of a subject, preferably, for treating a tooth lesion.
65. The kit of embodiment 64, wherein the tooth lesion is a caries lesion.
66. The kit of any of embodiments 64-65, wherein the tooth lesion is a subsurface lesion,
wherein the self-assembling peptide optionally is in dry, e.g., lyophilized form.
67. The kit of any of embodiments 64-65, wherein the tooth lesion is a cavitated lesion.
68. The kit of any of embodiments 64-65, wherein the tooth lesion is broken enamel or dentin, preferably, broken enamel.
69. The kit of any of embodiments 64-65 or 67 for use in pulp capping.
70. The kit of embodiment 64 for use in filling pits and/or fissures.
71. The kit of embodiment 64 for use in treating tooth sensitivity.
72. The kit of embodiment 64-67 or 71, wherein the treated region comprises a plurality, preferably, all teeth of a subject regardless of a diagnosis of active caries.
73. The kit of embodiment 64-72, wherein the kit comprises calcium phosphate particles having a size of 20 nm-5000 nm, optionally, 100-1000 nm.
74. The kit of any of embodiments 1-61 for use in inducing regeneration in a bone of a subject.
75. The kit of embodiment 74 for use in treating a bone defect, optionally, caused by a tumor or by trauma.
76. The kit of embodiment 74 for use in augmentation or reconstructive treatment of the alveolar ridge.
77. The kit of embodiment 74 for use in filling of a periodontal defect.
78. The kit of embodiment 74 for use in filling of a defect after root resection, apicoectomy, cystectomy or filling of an extraction socket to enhance preservation of the alveolar ridge.
79. The kit of embodiment 74 for use in elevation of the maxillary sinus floor.
80. The kit of embodiment 74 for use in filling of a periodontal defect.
81. The kit of embodiment 74 for use in filling of a peri-implant defect.
82. The kit of embodiment 74 for use in fixing an implant, e.g., a joint implant or cage.

83. The kit of embodiment 74 for use in spinal fusion.

84. The kit of embodiment 74-83, wherein the kit comprises calcium phosphate particles having a size of 1 $\mu$m-50 mm, optionally, 100-1000 $\mu$m.

85. Use of the kit of any of claims 1-83 for accelerating tooth remineralization or bone regeneration.

86. A method of treatment, comprising administering the components of the kit of any of claims 1-84 to a tooth or bone of a subject in need thereof.

87. The method of embodiment 86, wherein the composition comprising the self-assembling peptide is administered first.

88. The method of claim 86, wherein the composition comprising the self-assembling peptide is administered second.

89. The method of claim 86, wherein the components of the kit are administered essentially simultaneously.

## Detailed description of the drawings

Fig. 1 is a table summarizing the conditions and results of Example 1. n.a.: not applicable

Fig. 2 shows SEM pictures of crystals, or, if no crystals were formed, of dried solutions formed in the experiments of Example 1. A: HA (control) B: ACP (control), C: CaNa, D: CaPaNa, E: CaPaNaF, F: alpha, G: beta, H: gamma-1, I: gamma-2, J: delta.

a/b) different scales as specified

c) photo of solution formed by mixing

d) microscopic picture of crystals formed, as seen by a digital magnifying glass, if applicable.

## Examples

### Example 1

Individual solutions of $CaCl_2$ (calcium chloride dihydrate, pH 6) and $Na_2HPO_4$ (di-Sodium Phosphate Dihydrate, pH 8) were prepared at different concentrations and in the presence or absence of self-assembling peptide $P_{11}$-4 (Bachem, E104) and/or NaF. pH was corrected to 8, if required, with NaOH. Remineralisation solution (also known as artificial saliva) was prepared according to Kirkham et al. 2007, consisting of 1.5mM $Ca(NO_3)_2$, 0.9mM $KH_2PO_4$, 130mM KCl, 60 mM Tris (pH 7.4). All salts were obtained from Sigma Aldrich.

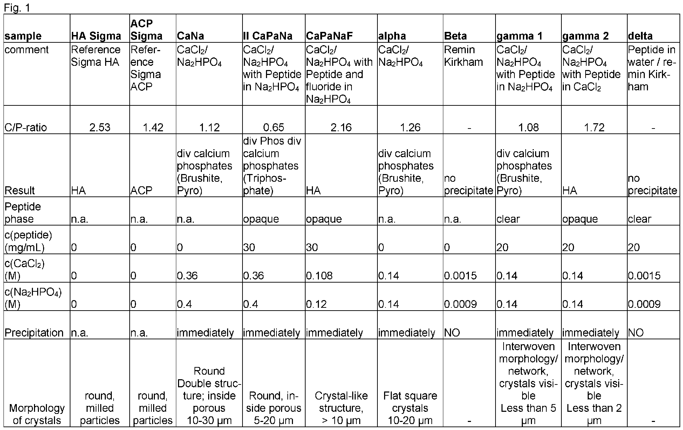

The individual compositions, as shown in Fig. 1, were transferred to a chamber of a dual chamber syringe (Sulzer Mixpac), the mixing unit, a static mixer, e.g. 1:1, DN2, brown (Sulzer Mixpac) added, and the compositions extruded. The concentrations relate to the concentration in the syringe if not specified otherwise. After extrusion, the respective concentrations are half of those specified, as each solution is diluted 1:1.

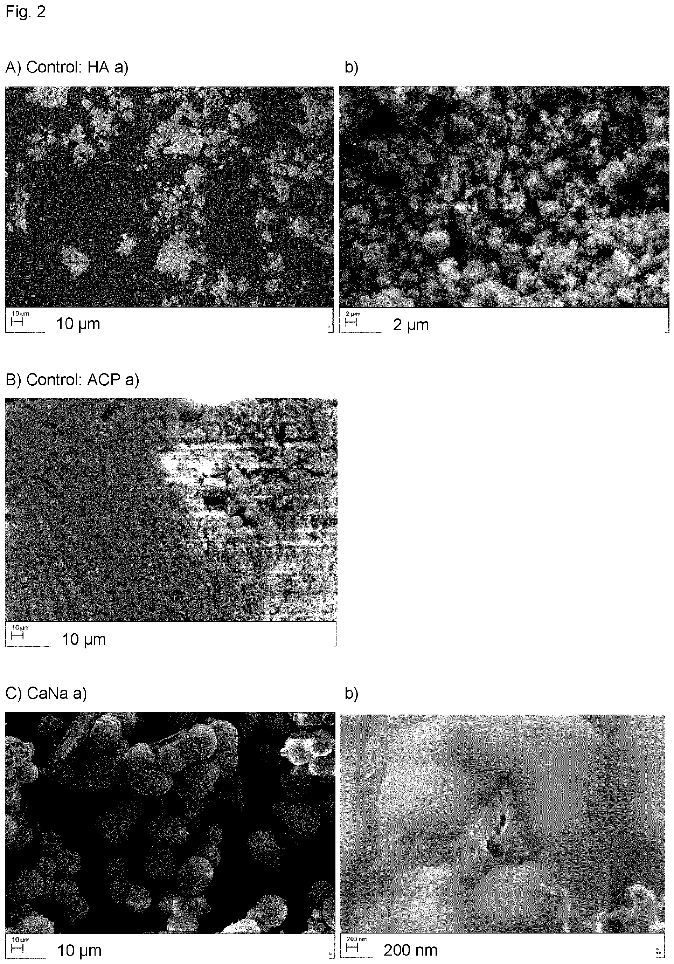

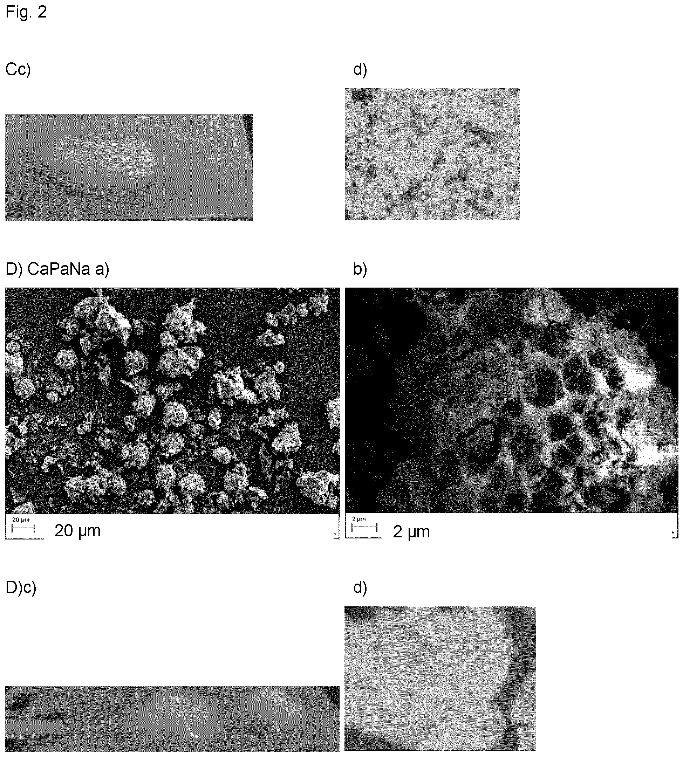

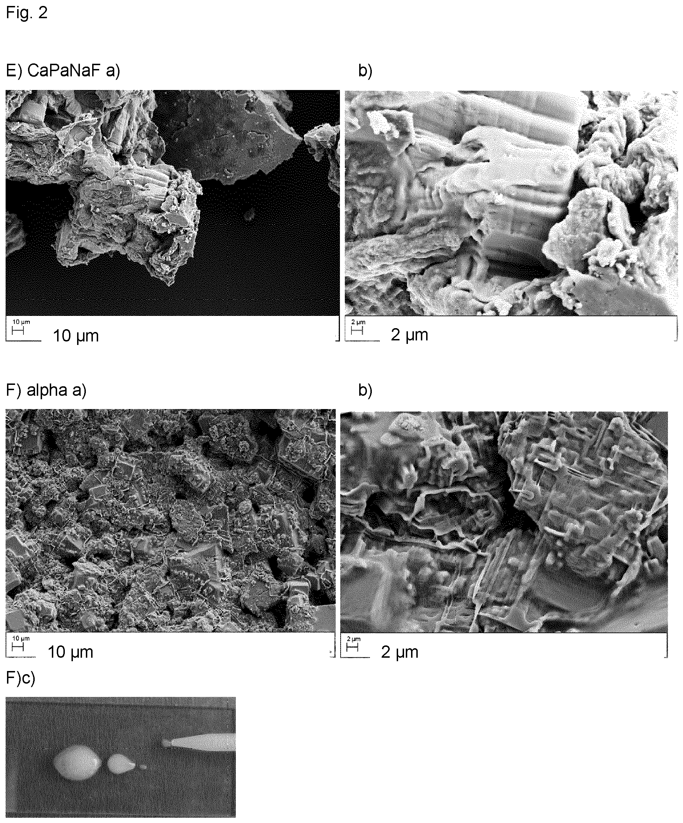

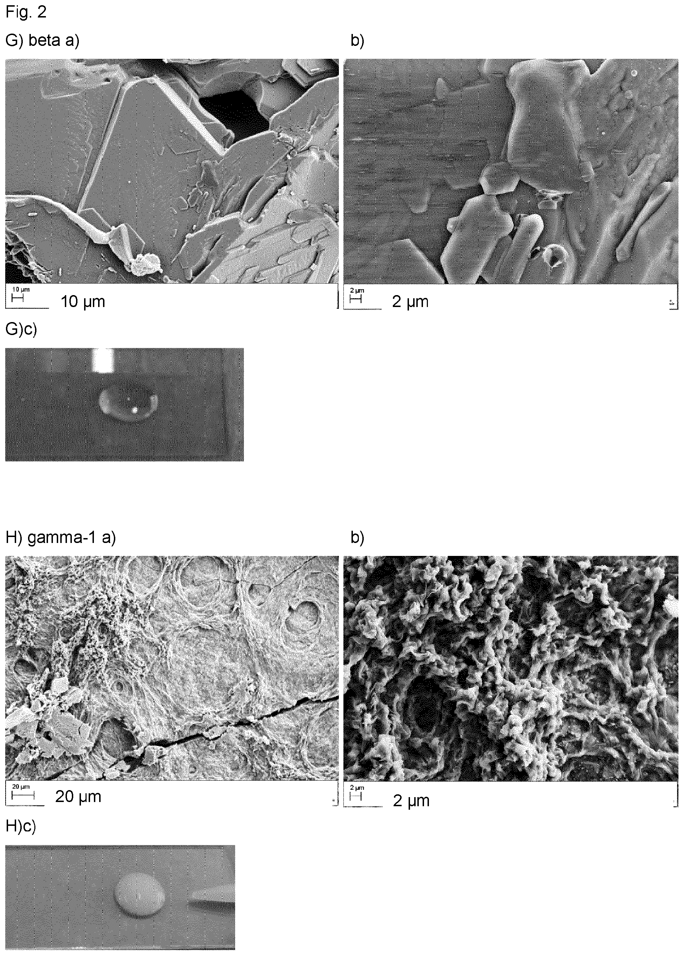

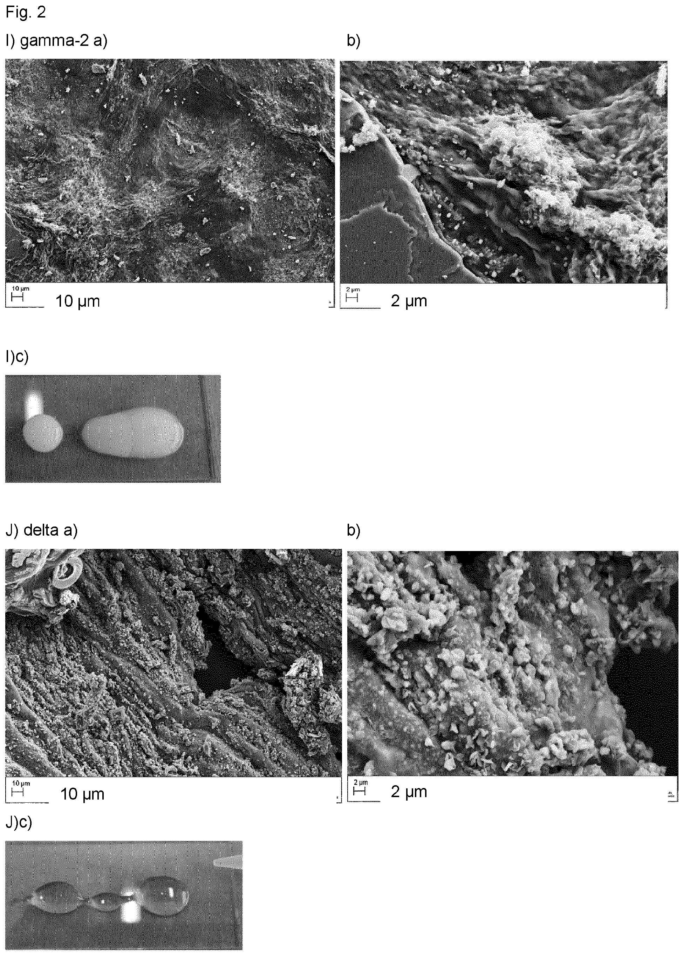

Further, with all compositions comprising sufficiently high concentrations of calcium and phosphate of at least 0.01 M, i.e., higher than the remineralisation buffer, calcium phosphate crystals precipitated, basically, immediately. The crystals, and for comparison, HA and ACP obtained from Sigma Aldrich, were analysed by SEM (Fig. 2) and by analysing the Ca/P ratio (Fig. 1), which allows for assessment of the calcium phosphate form formed. A Ca/P ratio of 1.6 or more is regarded as HA (e.g., Jeffre et al., 1993).

Further details of preparation and results for each experiment are provided below.

### Example 1A - SCaNa/CaPaNa/CaPaNaF:

Fresh solutions of 0.36M $CaCl_2$ (Calcium Chloride Dihydrate, Sigma Aldrich, pH 6) and 0.4M $Na_2HPO_4$ (di-Sodium Phosphate Dihydrate, Sigma Aldrich, pH 8) were prepared.

CaNa: The $CaCl_2$ solution and the $Na_2HPO_4$ solutions were each transferred into one chamber of the double chamber syringe system equipped with a static mixer (1:1). The two solutions were transferred through the mixer resulting in mixed liquids (pH 6-7), wherein a precipitate formed immediately. SEM showed spherical precipitate, wherein the particles were typical of ACP (cf. ACP control). The SEM further shows crystals typical of NaCl that were generated by the drying process.

CaPaNa: The self-assembling peptide $P_{11}$-4 was added to the $Na_2HPO_4$ solutions. 150mg self-assembling peptide $P_{11}$-4 was weighed into a 5mL Eppendorf tube and added to 3mL of $Na_2HPO_4$ solution (0.4M). pH was adjusted with NaOH (1N) to pH=8. The solution was sonicated (30s), yielding a clear and transparent solution having a P11-4 concentration of 30 mg/mL. This solution comprising the self-assembling peptide $P_{11}$-4 and $Na_2HPO_4$ and the $CaCl_2$ solution were each transferred to a chamber of the double chamber syringe system. The two solutions were transferred through the mixer 1:1, resulting in mixed liquids (pH 6-7), wherein a precipitate formed immediately. SEM showed spherical precipitate particles typical of ACP.

The addition of self-assembling peptide $P_{11}$-4 to the $Na_2HPO_4$ solution let to formation of calcium phosphate having a low Ca/P-ratio of 0.65, indicating still less crystalline character than without self-assembling peptide. The solution appears to be so highly supersaturated in $Ca^{2+}$ and $PO4^{3-}$-ions that precipitation occurs immediately, and addition of self-assembling peptide fibres cannot control/catalyse formation of more ordered crystals. Recrystallization on the self-assembling peptides may further lead to HA formation.

CaPaNaF: $Na_2HPO_4$ solution (0.108 M $Ca^{2+}$ and 0.12 M phosphate) containing 30 mg*mL$^{-1}$ self-assembling peptide $P_{11}$-4 was enriched with NaF (1450ppm Sodium Fluoride; i.e. the fluoride concentration of typical commercial toothpaste) to assess the influence of fluoride ions on the crystal growth. The resulting $Na_2HPO_4$ / self-assembling peptide $P_{11}$-4 / Fluoride and the $CaCl_2$ solution (0.108 M) were transferred to a double chamber syringe system and mixed 1:1. The liquid, having a pH or 6-7 and a final P11-4 concentration of 15 mg/mL, immediately showed 3D effects. Immediately, crystal-like structures formed that were similar to HA. The Ca/P ratio was >2. Thus, addition of fluoride at (1450ppm) favoured HA formation, even though the self-assembling peptide was favoured despite the self-assembling peptide $P_{11}$-4 being incorporated into the $CaCl_2$ solution.

**Example 1B - "greek" series**

**Investigating the influence of self-assembling peptide $P_{11}$-4 and ion concentrations.**

Fresh solutions of 0.14M $CaCl_2$ (pH=6-7; Calcium Chloride Dihydrate) and 0.14M $Na_2HPO_4$ (pH=8; di-Sodium Phosphate Dihydrate) and remineralisation buffer were prepared.

alpha: In the absence of self-assembling peptide, 0.14 M $CaCl_2$ solution and 0.14 M $Na_2HPO_4$ solution were transferred separately into the dual chamber syringe system equipped with a static mixer (1:1). The two solutions were transferred trough the mixer, resulting in immediately mixed, pH neutral liquids. Flat square crystals formed immediately. The crystals visible on the SEM appear to be NaCl cubic crystals. In between the cubic NaCl crystals, spheres of calcium phosphate precipitate can be seen, similar to in appearance to ACP. The Ca/P ratio was 1.26, also similar to ACP.

beta: Double concentrated remineralisation buffer/artificial saliva-solution was freshly prepared and immediately transferred one chamber of the dual chamber syringe, whereas in the other barrel, plain water was transferred. The two solutions were transferred trough the mixer, resulting in immediately mixed, pH neutral liquids. No precipitation or crystal formation occurred within the next 12 hours.

delta: Double concentrated remineralisation buffer/artificial saliva-solution was prepared as for experiment beta. However, self-assembling peptide $P_{11}$-4 dissolved in water at 20 mg/mL (pH adjusted to pH=8 with 1N NaOH), and sonicated for 30s, resulting in a homogenous, clear solution, was placed in the other chamber of the dual chamber syringe instead of water. The two solutions were transferred through the mixer resulting in immediately mixed, pH neutral liquids. No precipitation or crystal formation occurred within the next 12 hours. The crystals seen on the SEM picture only formed upon drying of the solution in the recording process.

gamma-1: The self-assembling peptide $P_{11}$-4 (20mg/mL) was added to 0.14M sodium phosphate solution (pH adjusted with 1 N NaOH to pH=8), sonicated for 30s, resulting in a clear solution. 0.14 M $CaCl_2$ solution and 0.14 M $Na_2HPO_4$ solution were transferred separately into the double chamber syringe and mixed 1:1. This resulted in pH neutral mixed liquids, wherein, immediately, a precipitate having an interwoven or network-like morphology precipitated, with crystals visible. The Ca/P-Ratio remained similar at Ca/P-Ratio=1.1. On the SEM pictures, it appears that self-assembling peptide $P_{11}$-4 fibres had formed, and that calcium phosphate precipitated around it.

gamma-2: The experiment was prepared as for gamma-1, however, the self-assembling peptide $P_{11}$-4 peptide was added into the 0.14M $CaCl_2$ solution at 20mg/mL (pH adjusted with 1 N NaOH to pH=8), resulting in a whitish/ opaque solution even after 30s sonication. The solutions were transferred separately into the dual chamber syringe and mixed 1:1. This resulted in pH neutral mixed liquids, wherein, immediately, a precipitate having an interwoven or network-like morphology precipitated, with crystals visible.

Surprisingly, when self-assembling peptide $P_{11}$-4 was incorporated into the $CaCl_2$ solution, the Ca/P-Ratio of the formed precipitate increased to Ca/P-Ratio=1.7, clearly indicating a formed apatite structure. The SEM pictures showed thin needle-type crystals around self-assembling peptide $P_{11}$-4 fibres.

In conclusion, addition of self-assembling peptide $P_{11}$-4 led to precipitation of calcium phosphate around the self-assembling peptide $P_{11}$-4 fibres when the concentration of $Ca^{2+}$ and $PO4^{3-}$ ions was in the range of physiological salt concentration, e.g., about 0.05-0.15 M. The crystalline form of the precipitated calcium phosphates depended on the presence of self-assembling peptide $P_{11}$-4, the concentration of $Ca^{2+}$ and $PO_4^{3-}$ ions, the presence of fluoride, and the solution in which self-assembling peptide $P_{11}$-4 was included prior to mixing.

If the concentration of calcium and phosphate ions was high, e.g., 0.36M, the precipitate tended to be amorphous both in absence and presence of self-assembling peptide $P_{11}$-4, indicating that the $Ca^{2+}$ and $PO_4^{3-}$ supersaturation was

so high that the precipitation reaction was faster than crystal nucleation (in other words, templating of the calcium phosphate crystals on the fibrilliar surface of self-assembling peptide was too slow to compete).

In the range of physiological salt concentration of $Ca^{2+}$ and $PO_4^{3-}$ ions (0.14M, or, in the resulting solution, 0.07 M), the precipitation reaction and the nucleation and secondary crystal growth seem to compete with regard to the formation of calcium phosphates. If the self-assembling peptide is included in the phosphate containing solution, ACP was formed, indicating that no templating nor prepositioning of $Ca^{2+}$ ions occurs prior to precipitation. However, when the self-assembling peptide is included in the $Ca^{2+}$ containing solution, hydroxyapatite was formed. Without intending to be bound by the theory, this may indicate that calcium ions were prepositioned, probably by being bound to low order aggregates of the self-assembling peptide, leading to fast nucleation of HA and secondary crystal growth, dominating over precipitation of ACP.

Interestingly, the addition of fluoride ions to the system resulted in formation of HA, even if the self-assembling peptide was included in the $Na_2HPO_4$ solution. It seems that presence of fluoride-ions favours nucleation of HA crystals.

**Sample Preparation for Microscopic Evaluation**

The samples were dried (12h; 40°C), rinsed - if possible - and dried again. The resulting precipitates were collected for scanning electron microscope (SEM) and Energy-dispersive X-ray spectroscopy (EDX) investigation.

Samples were individually placed on sample holders equipped with carbon tape and sputtered with gold (30s). The coating had an average thickness of 3-4nm. Following sputtering, samples were transferred to an SEM (SEM Supra 40 VP; 10 kV; WD=∼8 mm, Zeiss) and visually assessed.

The atomic composition was investigated using the EDX beam installed on the SEM. The standard magnification was defined with 1000x.

**Calculation of Ca/P**

EDX: The % element ratio was determined by critically assessing the Ca and P-peak using software (Thermoscientific NSS, Version 3.3). For calculation purposes, the Au (from the gold coating) was deducted in the calculations to avoid overestimation of the P-ratio. The calculation was as follows:

$$(\text{Element wt (Ca)} / \text{Element wt (P)}) = \text{Ca/P ratio.}$$

**Claims**

1.  A kit comprising,

    a) a self-assembling peptide comprising the amino acid sequence SEQ ID NO: 1 or an amino acid sequence having at least 80% identity thereto, and
    b)

(i) calcium and phosphate ions in separate compositions suitable for immediately forming calcium phosphate precipitates, preferably, amorphous calcium phosphate (ACP), if the compositions are mixed in the presence of water, wherein the compositions optionally are solutions, or
(ii) calcium phosphate particles, optionally, in the form of a suspension of calcium phosphate particles comprising at least 50% ACP.

2. The kit of claim 1 for use in inducing tooth remineralization or bone regeneration in at least a region of a subject in need thereof, wherein said remineralisation or regeneration is accelerated compared the absence of said calcium and phosphate ions.

3. The kit of any of the preceding claims, comprising
b) calcium and phosphate ions in separate compositions suitable for immediately forming calcium phosphate precipitates, ACP if the compositions are mixed in the presence of water, wherein the compositions preferably are solutions.

4. The kit of claim 3, wherein the solution comprising calcium ions comprises $Ca^{2+}$ in a concentration of 0.01-0.5 M, e.g., 0.1-0.15 M, wherein the solution preferably comprises $CaCl_2$.

5. The kit of any of claims 3 or 4, wherein the solution comprising phosphate ions comprises phopsphate in a concentration of 0.01-0.5 M, e.g., 0.1-0.15 M, wherein the solution preferably comprises $Na_2HPO_4$.

6. The kit of any of the claims 3-5, wherein the self-assembling peptide is a part of the composition comprising calcium ions, wherein the composition preferably is a solution.

7. The kit of any of the claims 3-5, wherein the self-assembling peptide is a part of the composition comprising phosphate ions, wherein the composition preferably is a solution.

8. The kit of any of the preceding claims, further comprising fluoride ions.

9. The kit of any of claims 1 - 2 or 8, comprising
b) calcium phosphate particles, preferably, comprising at least 50% ACP, optionally, an aqueous suspension of ACP.

10. The kit of any of the preceding claims, wherein the self-assembling peptide comprises the amino acid sequence SEQ ID NO: 1, wherein the self-assembling peptide optionally consists of said amino acid sequence.

11. The kit of any of the preceding claims, wherein the self-assembling peptide is provided in a composition selected from the group consisting of a dry composition, a lyophilized composition, a water-based solution, a toothpaste, a tooth gel, a mouthwash, a mouth spray, an oral care foam, a chewing gum, a toffee, a lozenge, or a candy.

12. The kit of any of claims 1-2 or 8-11, wherein the composition comprising the calcium phosphate particles is provided as a composition selected from the group consisting of a dry powder, a water-based suspension, a water-free product, a toothpaste, a tooth gel, a mouthwash, a mouth spray, an oral care foam, a chewing gum, a toffee, a lozenge, a tablet or a candy.

13. The kit of any of the preceding claims that is a solid or semi-solid composition comprising,

a) in a first part, the self-assembling peptide, and
b) in a second part, which is optionally surrounded by the first part, the phosphate ions or the calcium phosphate particles.

14. The kit of any of the preceding claims for use in medicine.

15. The kit of any of the preceding claims for use in inducing remineralization in or on a tooth of a subject, preferably, for treating a tooth lesion.

16. The kit of any of the preceding claims for use in inducing regeneration in a bone of a subject,
preferably, for treating a bone defect, optionally, caused by a tumor or by trauma, augmentation or reconstructive treatment of the alveolar ridge, filling of a periodontal defect, filling of a defect after root resection, apicoectomy, cystectomy, filling of an extraction socket to enhance preservation of the alveolar ridge, elevation of the maxillary sinus floor; filling of a periodontal defect, filling of a peri-implant defect or for orthopaedic indications.

Fig. 1

| sample | HA Sigma | ACP Sigma | CaNa | II CaPaNa | CaPaNaF | alpha | Beta | gamma 1 | gamma 2 | delta |
|---|---|---|---|---|---|---|---|---|---|---|
| comment | Reference Sigma HA | Reference Sigma ACP | $CaCl_2$/$Na_2HPO_4$ | $CaCl_2$/$Na_2HPO_4$ with Peptide in $Na_2HPO_4$ | $CaCl_2$/$Na_2HPO_4$ with Peptide and fluoride in $Na_2HPO_4$ | $CaCl_2$/$Na_2HPO_4$ | Remin Kirkham | $CaCl_2$/$Na_2HPO_4$ with Peptide in $Na_2HPO_4$ | $CaCl_2$/$Na_2HPO_4$ with Peptide in $CaCl_2$ | Peptide in water / remin Kirkham |
| C/P-ratio | 2.53 | 1.42 | 1.12 | 0.65 | 2.16 | 1.26 | - | 1.08 | 1.72 | - |
| Result | HA | ACP | div calcium phosphates (Brushite, Pyro) | div Phos div calcium phosphates (Triphosphate) | HA | div calcium phosphates (Brushite, Pyro) | no precipitate | div calcium phosphates (Brushite, Pyro) | HA | no precipitate |
| Peptide phase | n.a. | n.a. | n.a. | opaque | opaque | n.a. | n.a. | clear | opaque | clear |
| c(peptide) (mg/mL) | 0 | 0 | 0 | 30 | 30 | 0 | 0 | 20 | 20 | 20 |
| c($CaCl_2$) (M) | 0 | 0 | 0.36 | 0.36 | 0.108 | 0.14 | 0.0015 | 0.14 | 0.14 | 0.0015 |
| c($Na_2HPO_4$) (M) | 0 | 0 | 0.4 | 0.4 | 0.12 | 0.14 | 0.0009 | 0.14 | 0.14 | 0.0009 |
| Precipitation | n.a. | n.a. | immediately | immediately | immediately | immediately | NO | immediately | immediately | NO |
| Morphology of crystals | round, milled particles | round, milled particles | Round Double structure; inside porous 10-30 µm | Round, inside porous 5-20 µm | Crystal-like structure, > 10 µm | Flat square crystals 10-20 µm | - | Interwoven morphology/network, crystals visible Less than 5 µm | Interwoven morphology/network, crystals visible Less than 2 µm | - |

Fig. 2

A) Control: HA a)                                    b)

10 µm                                                2 µm

B) Control: ACP a)

10 µm

C) CaNa a)                                           b)

10 µm                                                200 nm

Fig. 2

Cc)

d)

D) CaPaNa a)

20 μm

b)

2 μm

D)c)

d)

Fig. 2

E) CaPaNaF a)                                    b)

10 µm                                             2 µm

F) alpha a)                                       b)

10 µm                                             2 µm

F)c)

Fig. 2

G) beta a)                                                    b)

10 µm                                                          2 µm

G)c)

H) gamma-1 a)                                                 b)

20 µm                                                          2 µm

H)c)

Fig. 2

I) gamma-2 a)

b)

I)c)

J) delta a)

b)

J)c)

Europäisches
Patentamt

European
Patent Office

Office européen
des brevets

## EUROPEAN SEARCH REPORT

Application Number

EP 20 20 2539

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 049 054 A1 (CREDENTIS AG [CH]) 3 August 2016 (2016-08-03) * paragraphs [0001], [0017], [0019], [0020], [0024], [0033], [0037]; claims; examples * | 1-16 | INV. A61K8/02 A61K8/24 A61Q11/00 A61K33/42 A61K33/16 A61K8/64 A61K6/75 |
| X | EP 2 853 256 A1 (CREDENTIS AG [CH]) 1 April 2015 (2015-04-01) * paragraphs [0001], [0020], [0021], [0025], [0032]; claims; examples * | 1-16 | |
| X | EP 3 248 590 A1 (CREDENTIS AG [CH]) 29 November 2017 (2017-11-29) * paragraphs [0001], [0053], [0079], [0091], [0105] - [0107]; claims; examples * | 1-16 | |
| X | DOBERDOLI DAFINA ET AL: "Abstract", SCIENTIFIC REPORTS, vol. 10, no. 1, 1 March 2020 (2020-03-01), XP055789537, DOI: 10.1038/s41598-020-60815-8 Retrieved from the Internet: URL:http://www.nature.com/articles/s41598-020-60815-8> | 1-12 | TECHNICAL FIELDS SEARCHED (IPC) A61K A61P A61Q |
| Y | * abstract * * page 1, last paragraph - page 2, paragraph first * | 1-16 | |
| Y | EP 2 698 162 A1 (CREDENTIS AG [CH]) 19 February 2014 (2014-02-19) * paragraphs [0001], [0002], [0016], [0029], [0033], [0035]; claims; examples * | 1-16 | |

-/--

The present search report has been drawn up for all claims

| Place of search | Date of completion of the search | Examiner |
|---|---|---|
| Munich | 24 March 2021 | Heller, Dorothée |

EPO FORM 1503 03.82 (P04C01)

Europäisches
Patentamt

European
Patent Office

Office européen
des brevets

## EUROPEAN SEARCH REPORT

Application Number

EP 20 20 2539

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 3 763 348 A1 (CREDENTIS AG [CH]) 13 January 2021 (2021-01-13) * paragraphs [0001], [0016], [0035], [0037], [0038], [0048], [0051], [0080], [0104]; claims; examples * ----- | 1-16 | |

TECHNICAL FIELDS
SEARCHED       (IPC)

The present search report has been drawn up for all claims

| Place of search | Date of completion of the search | Examiner |
|---|---|---|
| Munich | 24 March 2021 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 2539

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2021

| Patent document cited in search report | | Publication date | Patent family member(s) | | Publication date |
|---|---|---|---|---|---|
| EP 3049054 | A1 | 03-08-2016 | AU | 2014327234 A1 | 25-02-2016 |
| | | | BR | 112016000077 B1 | 22-12-2020 |
| | | | CA | 2915962 A1 | 02-04-2015 |
| | | | CN | 105555255 A | 04-05-2016 |
| | | | EP | 3049054 A1 | 03-08-2016 |
| | | | ES | 2669548 T3 | 28-05-2018 |
| | | | HK | 1218870 A1 | 17-03-2017 |
| | | | JP | 6649251 B2 | 19-02-2020 |
| | | | JP | 2016533325 A | 27-10-2016 |
| | | | PL | 3049054 T3 | 31-08-2018 |
| | | | RU | 2016115635 A | 30-10-2017 |
| | | | SG | 11201600874Y A | 28-04-2016 |
| | | | US | 2016199283 A1 | 14-07-2016 |
| | | | WO | 2015044268 A1 | 02-04-2015 |
| EP 2853256 | A1 | 01-04-2015 | NONE | | |
| EP 3248590 | A1 | 29-11-2017 | BR | 112018073866 A2 | 26-02-2019 |
| | | | CA | 3020489 A1 | 30-11-2017 |
| | | | CN | 109475484 A | 15-03-2019 |
| | | | EP | 3248590 A1 | 29-11-2017 |
| | | | EP | 3463279 A1 | 10-04-2019 |
| | | | JP | 2019516760 A | 20-06-2019 |
| | | | PT | 3463279 T | 04-09-2020 |
| | | | SG | 11201808904S A | 28-12-2018 |
| | | | US | 2019142724 A1 | 16-05-2019 |
| | | | WO | 2017202940 A1 | 30-11-2017 |
| EP 2698162 | A1 | 19-02-2014 | BR | 112015001782 A2 | 28-11-2017 |
| | | | CN | 104684573 A | 03-06-2015 |
| | | | CN | 110448677 A | 15-11-2019 |
| | | | EP | 2698162 A1 | 19-02-2014 |
| | | | EP | 2884991 A1 | 24-06-2015 |
| | | | ES | 2674159 T3 | 27-06-2018 |
| | | | HK | 1206998 A1 | 22-01-2016 |
| | | | JP | 6518588 B2 | 22-05-2019 |
| | | | JP | 2015526438 A | 10-09-2015 |
| | | | PL | 2884991 T3 | 28-09-2018 |
| | | | US | 2015232511 A1 | 20-08-2015 |
| | | | US | 2018340010 A1 | 29-11-2018 |
| | | | WO | 2014027012 A1 | 20-02-2014 |
| EP 3763348 | A1 | 13-01-2021 | EP | 3763348 A1 | 13-01-2021 |
| | | | WO | 2021005153 A1 | 14-01-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014027012 A1
- EP 2020069360 W
- WO 2017168183 A1
- WO 0006108 A1
- WO 2010042754 A2
- WO 02094204 A1
- WO 2017123986 A1
- US 20140186273 A1
- US 20170007737 A1
- WO 2004007532 A2
- WO 2014027012 A
- EP 1762215 A1
- US 20050037948 A1
- US 20080075675 A1
- US 20100247589 A1
- US 20100297203 A1
- WO 2007137606 A1
- WO 2013068020 A1

### Non-patent literature cited in the description

- **RUAN et al.** *Acta Biomater.,* 2013, vol. 9 (7), 7289-97
- **RUAN et al.** *J Vis Exp.,* 2014, vol. 10 (89
- **SCHMIDLIN et al.** *J Appl Oral Sci.,* 2016, vol. 24 (1), 31-36
- **ALKILZY et al.** *Adv Dent Res.,* 2018, vol. 29 (1), 42-47
- **BRUNTON et al.** *Br Dent J,* 2013, vol. 215, E6
- **KIND et al.** *J Dent Res.,* 2017, vol. 96 (7), 790-797
- **KIRKHAM et al.** *J Dent Res.,* 2007, vol. 86, 426-430
- **WEIR et al.** *J Dent Res.,* 2012, vol. 91 (10), 979-984
- **MEYER et al.** *Open Dent J.,* 2018, vol. 12, 406-423
- **XIAO et al.** *Dent. Mater.,* 2017, vol. 33 (11), 1217-1228
- **MAUDE.** *Soft matter,* 2011, vol. 7, 8085-8098
- **CARRICK.** *Tetrahedron,* 2008, vol. 63, 7457-7467
- *Compendium CE2,* vol. 25 (1
- **COMES et al.** *Acta Biomaterialia,* 2010, vol. 6 (9), 3362-3378
- **JEFFRE et al.** *Marine and Freshwater Research,* 1993, vol. 44, 609-634
- **ROVERI, BATTISTELLI et al.** Nanocomposites for Engineering Applications. *J. Nanomater.,* 2009, 1-9
- **THOMSON.** *Caries Res,* 2014, vol. 48, 411